# EUROPEAN PATENT APPLICATION

(11) **EP 4 541 342 A1**
(43) Date of publication of application: **23.04.2025**
(21) Application number: 23846890.4
(22) Date of filing: 18.07.2023
(51) Int. Cl.: A61K 8/06, A61K 8/81, A61K 8/34, A61Q 19/00

(54) **SELF-EMULSIFIED EMULSION COSMETIC COMPOSITION**

(30) Priority: 28.07.2022 KR 20220093695
(71) Applicant: Kolmar Korea Co., Ltd., Sejong 30004 (KR)
(72) Inventor: BAE, Hyung Jin, Seoul 06800 (KR); KIM, Jin Mo, Seoul 06800 (KR); KIM, Jin Young, Seoul 06800 (KR); JEONG, Eun Sook, Seoul 06800 (KR); CHOI, Eun Sol, Seoul 06800 (KR)
(74) Representative: Nederlandsch Octrooibureau
(86) International application number: PCT/KR2023/010302
(87) International publication number: WO 2024/025231

(57) **Abstract**

The present invention relates to an emulsion cosmetic composition having excellent stability.

## Description

### Technical Field

The present invention relates to an emulsion cosmetic composition having excellent stability.

### Background Art

Various types of emulsion cosmetics are used to provide various active ingredients to the skin, and emulsion type cosmetics are generally classified into an oil-in-water (O/W) type and a water-in-oil (W/O) type. These all contain oil and aqueous phases, and thus have the advantage of providing softening and moisturizing effects. Various technologies have been developed to improve the properties of O/W type or W/O type cosmetic compositions, such as formulation stability, and for example, Korean Patent Publication No. 1996-001690 discloses a W/O/W type cosmetic composition including a W/O formulation containing a perfluoropolymethylisopropyl ether base. However, conventional emulsion-type formulations have a problem in that, when an unstable active ingredient is contained among active ingredients, the formulation is easily separated or the active ingredient precipitates in the formulation.

To increase the stability of such emulsion-type cosmetic compositions, high-content surfactants are usually used, but these surfactants have the problem of being against current consumer trends that consider environmental friendliness and skin safety. Therefore, there is a demand for the development of an emulsion cosmetic composition having excellent interfacial stability between the aqueous phase and the oil phase without containing a surfactant.

### [Prior Art Documents]

(Patent Document 1) Korean Patent Publication No. 1996-001690

### DISCLOSURE

### Technical Problem

The present invention provides an emulsion cosmetic composition that exhibits excellent stability without containing a surfactant.

### Technical Solution

An emulsion cosmetic composition of the present invention including: an aqueous phase containing a self-emulsifying agent and a self-emulsifying stabilizer; and an oil phase containing an oil phase stabilizer.

### Advantageous Effects

The present invention provides an emulsion cosmetic composition that exhibits excellent stability due to high interfacial stability between the aqueous phase and the oil phase without containing a surfactant. The cosmetic composition according to the present invention contains no surfactant, and thus causes little skin irritation and is environmentally friendly. In addition, as the cosmetic composition is an emulsion formulation, it has excellent spreadability and feeling of use, and also has excellent makeup durability and water resistance. In addition, the cosmetic composition of the present invention is suitable for stabilizing an active ingredient such as retinol within the formulation.

### Brief Description of Drawings

FIG. 1 depicts photographs the results of observing of the emulsion stability of Examples 1 to 3 and Comparative Examples 1 to 4.
FIG. 2 depicts photographs the results of observing the retinol stability of Examples 1 to 3 and Comparative Examples 1 to 4.

### Best Mode

Hereinafter, the present invention will be described. However, the present invention is not limited only by the following description, and each component may be variously modified or selectively used as needed. Therefore, it should be understood that the present invention encompasses all modifications, equivalents, or substitutes within the spirit and scope of the present invention.

The present invention relates to an emulsion cosmetic composition including: an aqueous phase containing a self-emulsifying agent and a self-emulsifying stabilizer; and an oil phase containing an oil phase stabilizer.

### Aqueous Phase

The aqueous phase of the emulsion cosmetic composition according to the present invention contains a self-emulsifying agent and a self-emulsifying stabilizer. The aqueous phase may further contain a thickener, a pH buffer, an active ingredient, and additives commonly used in the art.

According to the present invention, it is possible to provide an emulsion cosmetic composition having excellent stability without using a surfactant by using a self-emulsifying agent that induces self-emulsification and ensures the stability of the formulation. The self-emulsifying agent is a polymer having a hydrophobic alkyl chain, and may include, for example, at least one selected from the group consisting of an acrylates/palmeth-25 copolymer, an acrylates/aminoacrylates/C10-30 alkyl PEG-20 itaconate copolymer, an acrylates/beheneth-25 methacrylate copolymer, an acrylates/beheneth-25 methacrylate/steareth-30 methacrylate copolymer, an acrylates/C10-30 alkyl acrylate crosspolymer, an acrylates/C10-30 alkyl methacrylate copolymer, an acrylates/C12-13 alkyl methacrylates/methoxyethyl acrylate crosspolymer, an acrylates/C12-22 alkyl methacrylate copolymer, an acrylates/C26-29 olefin copolymer, an acrylates/C5-8 alkyl acrylate copolymer, an acrylates/ceteareth-20 methacrylate crosspolymer, an acrylates/ceteareth-25 methacrylate/methacrylamide crosspolymer, an acrylates/ceteth-20 itaconate copolymer, an acrylates/ceteth-20 methacrylate copolymer, an acrylates/laureth-25 methacrylate copolymer, an acrylates/palmeth-25 acrylate copolymer, an acrylates/palmeth-25 itaconate copolymer, an acrylates/steareth-20 itaconate copolymer, an acrylates/steareth-20 methacrylate copolymer, an acrylates/steareth-20 methacrylate crosspolymer, an acrylates/steareth-50 acrylate copolymer, an acrylates/steareth-30 methacrylate copolymer, an acrylic acid/C12-22 alkyl acrylate copolymer, an acrylates/beheneth-25 methacrylate/HEMA crosspolymer, an ammonium acryloyldimethyltaurate/beheneth-25 methacrylate crosspolymer, an ammonium acryloyldimethyltaurate/laureth-7 methacrylate copolymer, an ammonium acryloyldimethyltaurate/steareth-8 methacrylate copolymer, an AMP-acrylate/C1-18 alkyl acrylates/C1-8 alkyl acrylamide copolymer, an AMP-acrylates/C1-18 alkyl acrylate/C1-8 alkyl acrylamide copolymer, an AMP-acrylates/C1-18 alkyl acrylates/C1-8 alkyl acrylamide/hydroxyethylacrylate copolymer, a butyl acrylate/C6-14 perfluoroalkylethyl acrylate/mercaptopropyl dimethicone copolymer, a C12-22 alkyl acrylate/hydroxyethylacrylate copolymer, a C18-22 alkyl PEG-25 methacrylate/diethylaminoethyl methacrylate copolymer, a C20-24 olefin/oleyl alcohol copolymer, a C26-28 alkyldimethylsilyl polypropylsilsesquioxane, a C30-38 olefin/isopropyl maleate/MA copolymer, a C4-6 olefin/styrene copolymer, a C6-14 perfluoroalkylethyl acrylate/HEMA copolymer, a C8-22 alkyl acrylates/butyl dimethicone methacrylate copolymer, a C8-22 alkyl acrylates/methacrylic acid crosspolymer, a potassium acrylates/C10-30 alkyl acrylate crosspolymer, a sodium acrylates/beheneth-25 methacrylate crosspolymer, and a sodium acrylates/C10-30 alkyl acrylate crosspolymer, without being limited thereto. For example, the self-emulsifying agent may include at least one selected from the group consisting of an acrylates/C10-30 alkyl acrylate crosspolymer, an ammonium acryloyldimethyltaurate/beheneth-25 methacrylate crosspolymer, an acrylates/palmeth-25 acrylate copolymer, and an acrylates/beheneth-25 methacrylate copolymer.

The self-emulsifying agent may be contained in an amount of 0.05 to 5 wt% based on the total weight of the cosmetic composition. If the content of the self-emulsifying agent is lower than the lower limit of the above range, the composition may not be emulsified, or even if it is emulsified, the emulsion may not be maintained. If the content of the self-emulsifying agent is higher than the upper limit of the above range, the viscosity of the composition may become so high that emulsification is impossible, or even if the composition is emulsified, it may be difficult to use due to stickiness.

According to the present invention, it is possible to provide an emulsion cosmetic composition having excellent stability without using a surfactant by using a self-emulsifying stabilizer that stabilizes a self-emulsifying formulation. The self-emulsifying stabilizer is an amphiphilic substance, and may include, for example, a diol compound having two hydroxyl groups or a mixture thereof. For example, the self-emulsifying stabilizer may include at least one selected from the group consisting of 1,2-butanediol, 1,2-hexanediol, 1,2-pentanediol, 1,2-octanediol, 3-(2-ethylhexyloxy)propane-1,2-diol, 1,2-dodecanediol, propane-1,2-diol, 1,2-decanediol, 1,2-tetradecanediol, 1,2-hexadecanediol, 1,2-octadecanediol, 1,2-eicosanediol, 1,2-hexacosanediol, 2,3-butanediol, 2-methyl-1,3-propanediol, 1,2-octacosanediol, 1,5-pentanediol, 1,6-hexanediol, 1,9-nonanediol, 1,10-decanediol, 1,12-dodecanediol, 1,2-propanediol, 1,7-heptanediol, 1,8-octanediol, 1,14-tetradecanediol, 1,16-hexadecanediol, and 12-hydroxystearyl alcohol, without being limited thereto. For example, the self-emulsifying stabilizer may include at least one selected from the group consisting of 3-(2-ethylhexyloxy)propane-1,2-diol, 2,3-butanediol, 1,2-octanediol, 1,2-hexanediol, and 1,2-pentanediol.

The self-emulsifying stabilizer may be contained in an amount of 0.01 to 5 wt% based on the total weight of the cosmetic composition. If the content of the self-emulsifying stabilizer is lower than the lower limit of the above range, self-emulsifying particles may not be maintained, and if the content of the self-emulsifying stabilizer is higher than the upper limit of the above range, it may cause skin irritation.

The self-emulsifying agent and the self-emulsifying stabilizer may be mixed at a weight ratio of 1:0.002 to 100. If the mixing ratio between the self-emulsifying agent and the self-emulsifying stabilizer is lower than the lower limit of the above range, self-emulsification may not occur or the emulsion may not be maintained, and if the mixing ratio is higher than the upper limit of the above range, it may cause skin irritation or self-emulsification may not be maintained.

The aqueous phase may further contain a polyol. The polyol acts to provide skin softening and moisturizing effects, and may include, for example, at least one selected from the group consisting of polyethylene glycol, glycerin, dipropylene glycol, butylene glycol, pentylene glycol, methylpropanediol, sorbitol, diglycerin, erythritol, pentaerythritol, polybutylene glycol-10, polyglycerin-3, polyglycerin-4, polyglycerin-6, polyglycerin-10, polyglycerin-20, polyglycerin-40, sorbeth-5, sorbeth-6, sorbeth-20, sorbeth-30, sorbeth-40, inositol, maltitol, mannan, mannitol, mannose, lactitol, lactose, dihydroxypropyl PG-glucoside, dithiaoctanediol, fructose, glucamine, methylglucamine, glucose, 1,2,6-hexanethiol, methyl gluceth-10, methyl gluceth-20, ozonized glycerin, phytantriol, thioglycerin, threitol, trimethylolpropane, and xylitol, without being limited thereto.

The polyol may be contained in an amount of 1 to 20 wt% based on the total weight of the cosmetic composition. If the content of the polyol is lower than the lower limit of the above range, the moisturizing effect may be reduced, and if the content is higher than the upper limit of the above range, stickiness and irritation may occur.

The aqueous phase may further contain a thickener. The thickener serves to maintain the stability (sinking/floating) of the particles in the aqueous phase. The thickener may include at least one of an acrylic acid-based polymer and a polysaccharide-based polymer.

The acrylic acid-based polymer includes an acrylic acid polymer or an acrylic acid derivative polymer, and may include, for example, at least one selected from the group consisting of a vinylpyrrolidone-acrylic acid copolymer, a methyl methacrylate-acrylic acid copolymer, a methyl methacrylate-methacrylic acid copolymer, carbomer, sodium polyacrylate, polyacrylic acid (PAA), and polymethacrylic acid, without being limited thereto.

The polysaccharide-based polymer includes a polysaccharide or a polysaccharide derivative polymer, and may include, for example, at least one selected from the group consisting of sodium alginate, carrageenan, cellulose sulfate, dextrin sulfate, carboxymethyl cellulose, xanthan gum, locust bean gum, guar gum, carrageenan, sodium hyaluronate, and sulfate group-containing glycosaminoglycans, without being limited thereto.

The thickener may be contained in an amount of 0.05 to 10 wt%, for example, 0.1 to 5 wt%, based on the total weight of the cosmetic composition. If the content of the thickener is lower than the lower limit of the above range, the viscosity of the composition is low, and thus particle stability may be poor, and if the content is higher than the upper limit of the above range, the viscosity of the composition is high, and thus the feeling of use, such as spreadability and stickiness, may be poor, and it may be difficult to produce an emulsion.

The aqueous phase may further contain a pH buffer. The pH buffer may stabilize oil-soluble ingredients contained in the oil phase. The pH buffer may include at least one selected from the group consisting of malic acid, adipic acid, benzoic acid, lactic acid, citric acid, sodium hydroxide, hydrochloric acid, potassium chloride, potassium hydroxide, glycine, potassium hydrogen phthalate, sodium citrate, sodium acetate, acetic acid, fumaric acid, succinic acid, phosphoric acid, tartaric acid, ammonium bicarbonate, ammonium carbonate, ammonium phosphate, potassium bicarbonate, potassium carbonate, potassium borate, potassium citrate, potassium phosphate, sodium bicarbonate, sodium carbonate, sodium succinate, and sodium fumarate, without being limited thereto. For example, the pH buffer may include at least one selected from the group consisting of citric acid, sodium citrate, sodium hydroxide, and potassium hydroxide.

The pH buffer may be contained in an amount of 0.01 to 5 wt% based on the total weight of the cosmetic composition. If the content of the pH buffer is lower than the lower limit of the above range, it may be difficult to stabilize an active ingredient such as retinol, and if the content is higher than the upper limit of the above range, it may cause skin irritation or broke the emulsion.

The aqueous phase may further contain an active ingredient. The active ingredient is a hydrophilic material with excellent compatibility with purified water, and may include, for example, at least one selected from the group consisting of ascorbic acid, niacinamide, adenosine, hyaluronic acid, azelaic acid, kojic acid, glycolic acid, arbutin, peptides, zinc sulfate, glutathione, ferulic acid, quercetin, tocopherol, gallic acid, catechin, caffeic acid, rosmarinic acid, and kojic acid, without being limited thereto.

The active ingredient may be contained in an amount of 0.01 to 20 wt%, for example, 0.05 to 10 wt%, based on the total weight of the cosmetic composition. If the content of the active ingredient is lower than the lower limit of the above range, it may be difficult to expect the effect of the active ingredient on the skin, and if the content is higher than the upper limit of the above range, it may cause skin irritation.

The aqueous phase contains purified water in an amount making 100 wt% of the total weight of the cosmetic composition. For example, the purified water may be contained in an amount of 5 to 95 wt% based on the total weight of the cosmetic composition.

### Oil Phase

The oil phase of the emulsion cosmetic composition according to the present invention contains an oil phase stabilizer. The oil phase may further contain a self-emulsifying stabilizer, an active ingredient, and additives commonly used in the art.

The oil phase contains an oil phase stabilizer. The oil phase stabilizer maintains the emulsion stably by exerting a thickening effect and an effect of preventing breakage or aggregation of the particles formed of the aqueous phase or the particles formed of the oil phase. For example, the oil phase stabilizer may include at least one selected from the group consisting of amino acid derivatives such as N-lauroyl-L-glutamic acid, α,γ-di-n-butylamine, etc., dextrin fatty acid esters such as dextrin palmitate, dextrin stearate, dextrin palmitate/ethylhexanoate, dextrin myristate, etc., sucrose fatty acid esters such as sucrose palmitate, sucrose stearate, etc., fructooligosaccharide fatty acid esters such as inulin stearate, fructooligosaccharide 2-ethylhexanoate, etc., sorbitol benzylidene derivatives such as monobenzylidene sorbitol, dibenzylidene sorbitol, etc., polyurethane-79, polyethylene, glyceryl behenate, glyceryl behenate/eicosadioate, stearoyl inulin, trihydroxystearin, polybutene, polyamide-2, polyamide-3, polyamide-5, polyamide-8, hydrogenated vegetable oil, an isophorone diisocyanate (IPDI) copolymer, bentonite, hectorite, montmorillonite, attapulgite, sepiolite, calcium silicate, silica, silica silylate, silica dimethyl silylate, a polymethylsilsesquioxane/silica crosspolymer, mica, polymethyl methacrylate, nylon-6, iron oxides, tin oxide, a methyl methacrylate crosspolymer, titanium dioxide, synthetic fluorphlogopite, polymethylsilsesquioxane, an ethylene/propylene/styrene copolymer, and a butylene/ethylene/styrene copolymer, without being limited thereto.

The oil phase stabilizer may be contained in an amount of 0.5 to 80 wt%, for example, 1 to 40 wt%, based on the total weight of the cosmetic composition. If the content of the oil phase stabilizer is lower than the lower limit of the above range, the effect of enhancing high-temperature stability and long-term stability may be insufficient, and if the content is higher than the upper limit of the above range, the viscosity and hardness of the composition may increase, which may not only impair the feeling of use, but also make it difficult to produce an emulsion.

The oil phase may further contain a self-emulsifying stabilizer. By using the self-emulsifying stabilizer that stabilizes the self-emulsifying formulation, it is possible to provide an emulsion cosmetic composition having excellent stability without using a surfactant. As the self-emulsifying stabilizer, a material exemplified as the self-emulsifying stabilizer contained in the aqueous phase may be used.

The self-emulsifying stabilizer may be contained in an amount of 0.01 to 5 wt%, for example, 0.1 to 2.5 wt%, based on the total weight of the cosmetic composition. If the content of the self-emulsifying stabilizer is lower than the lower limit of the above range, the self-emulsifying particles may not be maintained, and if the content is higher than the upper limit of the above range, it may cause skin irritation.

The oil phase contains an oil. As the oil, any oil used in the art may be used without any special limitation. For example, the oil may include at least one selected from the group consisting of mineral oil, isohexadecane, isostearyl isostearate, isopropyl myristate, caprylic/capric triglyceride, diisopropyl adipate, isononyl isononanoate, isodecyl neopentanoate, cetyl ethyl hexanoate, octyldodecyl myristate, di-C12-13 alkyl malate, pentaerythrityl tetraisostearate, pentaerythrityl tetraethyl hexanoate, diisostearyl malate, isopropyl palmitate, C12-15 alkyl benzoate, dicaprylic carbonate, dicaprylic ether, 2-octyldodecanol, dimethicone, lauryl dimethicone, cyclopentasiloxane, cyclohexasiloxane, trisiloxane, methyl trimethicone, amodimethicone, phenyl trimethicone, phenyl methicone, dimethicone copolyol, dimethicone copolyol acetate, silicone glycol copolyol, dimethiconol, methyl polysiloxane, methylphenyl polysiloxane, dimethicone copolyol methyl ether, methylcyclo polysiloxane, dimethyl polysiloxane, hexamethyl disiloxane, aminopropyl dimethicone, caprylyl methyl polysiloxane, and simethicone, without being limited thereto.

The oil may be contained in an amount of 1 to 80 wt% based on the total weight of the cosmetic composition. If the content of the oil is lower than the lower limit of the above range, an emulsion-type formulation may not be formed, and if the content is higher than the upper limit of the above range, emulsion particles may not be formed.

The oil phase may further contain an active ingredient. For example, the active ingredient may include at least one selected from the group consisting of retinol, retinyl palmitate, retinal, tretinoin, retinoic acid, adapalene, tazarotene, isotretinoin, calciferol, tocopherol, and phytonadione, without being limited thereto.

The active ingredient may be contained in an amount of 0.01 to 5 wt%, for example, 0.02 to 2.5 wt%, based on the total weight of the cosmetic composition. If the content of the active ingredient is lower than the lower limit of the above range, it may not provide efficacy to the skin, and if the content is higher than the upper limit of the above range, it may cause skin irritation.

### Emulsion Cosmetic Composition

The emulsion cosmetic composition of the present invention includes the above-described aqueous phase and the above-described oil phase, and may be prepared as an oil-in-water (O/W) type, a water-in-oil (W/O) type, a water-in-oil-in-water (W/O/W) type, or an oil-in-water-in-oil (O/W/O) type.

The emulsion cosmetic composition of the present invention may be any one formulation selected from a lotion, essence, cream, serum, mask liquid, ampoule, or pack formulation, without being limited thereto. For example, the emulsion cosmetic composition of the present invention may be an emulsion containing retinol.

The emulsion cosmetic composition of the present invention may be prepared according to various methods known in the art. For example, it may be prepared by mixing the oil phase with the aqueous phase using a homomixer or an agi mixer.

### Mode for Invention

Hereinafter, the present invention will be described in more detail by way of examples. However, the following examples are only intended to facilitate the understanding of the present invention and should not be construed to limit the scope of the present invention in any way.

### [Examples 1 to 3]

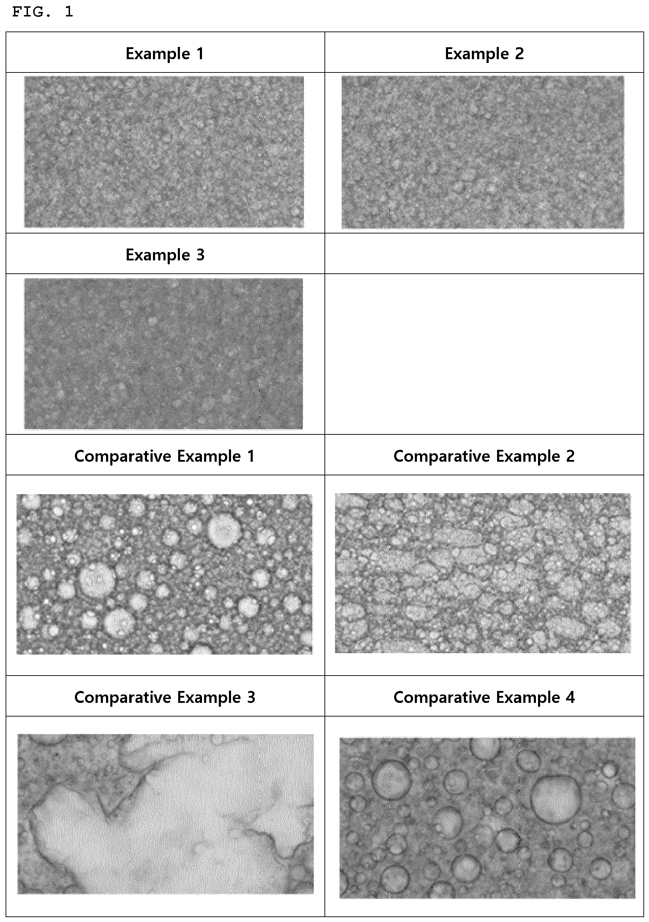

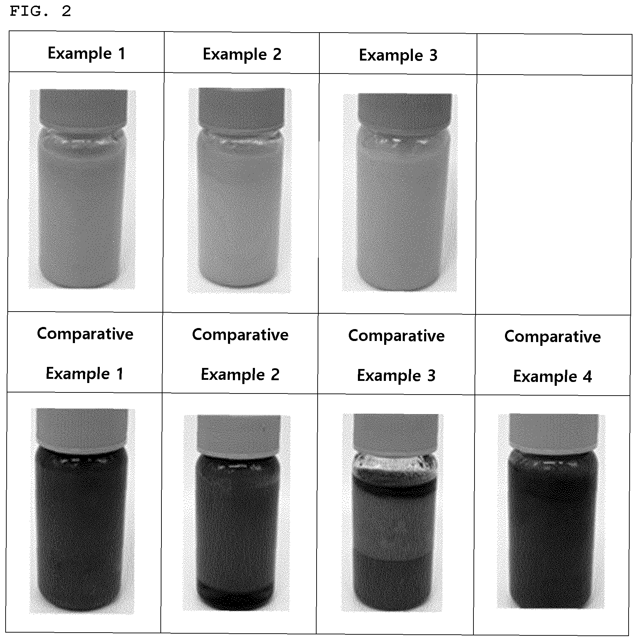

An O/W type emulsion composition of each Example was prepared according to the composition shown in Table 1 below, and then the physical properties thereof were measured according to the following method. The measured physical properties are shown in Table 1 below and FIGS. 1 and 2.

### Self-Emulsifying Ability

Particle formation was checked by microscopic observation.

### [Criteria for evaluation]

o: Particles were formed uniformly.
△: Particles were formed non-uniformly.
X: No emulsion particles were formed.

### Emulsion Stability

After the emulsion composition was left at 50°C for one month, the morphology of the O/W particles was observed under a microscope.

### [Criteria for evaluation]

∘: The particle size remained unchanged.
X: The particles were broken or aggregated and the particle size increased or decreased.

### Retinol Stability

After the emulsion composition was left at 50°C for one month, the color change thereof was observed.

### [Criteria for evaluation]

∘: No discoloration occurred.
X: Discolored.

### [Comparative Examples 1 to 4]

An O/W type emulsion composition of each Comparative Example was prepared according to the composition shown in Table 1 below, and the physical properties thereof were measured in the same manner as in the Examples. The measured physical properties are shown in Table 1 below and FIGS. 1 and 2.

**[Table 1]**

| Component (parts by weight) | | Example 1 | Example 2 | Example 3 | Comp. Example 1 | Comp. Example 2 | Comp. Example 3 | Comp. Example 4 |
|---|---|---|---|---|---|---|---|---|
| Aqueous phase part | Water | To 100 | To 100 | To 100 | To 100 | To 100 | To 100 | To 100 |
| | Polyol | 5 | 5 | 5 | 5 | 5 | 5 | 5 |
| | Thickener | 2 | 2 | 2 | 2 | 2 | 2 | 2 |
| | Self-emulsifying agent | 5 | 0.05 | 0.5 | 0.5 | 0.5 | - | 0.5 |
| | Self-emulsifying stabilizer | 0.01 | 5 | 0.5 | 10 | - | 0.5 | 0.5 |
| Oil phase part | Oil | 15 | 15 | 15 | 15 | 15 | 15 | 15 |
| | Oil phase stabilizer | 10 | 10 | 10 | 10 | 10 | 10 | - |
| | Retinol | 0.5 | 0.5 | 0.5 | 0.5 | 0.5 | 0.5 | 0.5 |
| Self-emulsifying ability | | ○ | ○ | ○ | Δ | X | X | O |
| Emulsion stability | | ○ | ○ | ○ | X | X | X | X |
| Retinol stability against discoloration | | ○ | ○ | ○ | X | X | X | X |

| | | | | | | | | |
|---|---|---|---|---|---|---|---|---|
| Polyol: glycerin Thickener: sodium alginate Self-emulsifying agent: acrylates/C10-30 alkyl acrylate crosspolymer Self-emulsifying stabilizer: 2,3-butanediol Oil: octyldodecanol Oil phase stabilizer: hectorite | | | | | | | | |

As shown in Table 1 above and FIGS. 1 and 2, the emulsion compositions of Examples 1 to 3 according to the present invention exhibited excellent emulsion stability through self-emulsification and also exhibited excellent retinol stability. On the other hand, in the case of the emulsion composition of Comparative Example 1, which contains the self-emulsifying stabilizer in an amount outside the range specified in the present invention, self-emulsification occurred insufficiently, the particle size increased, and discoloration of retinol was observed. In the case of the emulsion composition of Comparative Example 2, which does not contain the self-emulsifying stabilizer, and the emulsion composition of Comparative Example 3, which does not contain the self-emulsifying agent, self-emulsification did not occur, particles were not formed, and discoloration of retinol was observed. In the case of the emulsion composition of Comparative Example 4, which does not contain the oil stabilizer, self-emulsification occurred, but the particle size increased and discoloration of retinol was observed.

### Industrial Applicability

The present invention provides an emulsion cosmetic composition that exhibits excellent stability due to high interfacial stability between the aqueous phase and the oil phase without containing a surfactant. The cosmetic composition according to the present invention contains no surfactant, and thus causes little skin irritation and is environmentally friendly. In addition, as the cosmetic composition is an emulsion formulation, it has excellent spreadability and feeling of use, and also has excellent makeup durability and water resistance. In addition, the cosmetic composition of the present invention is suitable for stabilizing an active ingredient such as retinol within the formulation.

## Claims

1. An emulsion cosmetic composition comprising: an aqueous phase containing a self-emulsifying agent and a self-emulsifying stabilizer; and an oil phase containing an oil phase stabilizer,
wherein the self-emulsifying stabilizer is an amphiphilic substance, and
the self-emulsifying stabilizer is contained in an amount of 0.01 to 5 wt% based on the total weight of the cosmetic composition.

2. The emulsion cosmetic composition of claim 1, wherein the self-emulsifying agent is a polymer having a hydrophobic alkyl chain.

3. The emulsion cosmetic composition of claim 1, wherein the self-emulsifying agent comprises at least one selected from the group consisting of an acrylates/C10-30 alkyl acrylate crosspolymer, an ammonium acryloyldimethyltaurate/beheneth-25 methacrylate crosspolymer, an acrylates/palmeth-25 acrylate copolymer, and an acrylates/beheneth-25 methacrylate copolymer.

4. The emulsion cosmetic composition of claim 1, wherein the self-emulsifying stabilizer comprises a diol compound having two hydroxyl groups or a mixture thereof.

5. The emulsion cosmetic composition of claim 1, wherein the self-emulsifying stabilizer comprises at least one selected from the group consisting of 3-(2-ethylhexyl)oxylpropane-1,2-diol, 2,3-butanediol, 1,2-octanediol, 1,2-hexanediol, and 1,2-pentanediol.

6. The emulsion cosmetic composition of claim 1, comprising, based on the total weight of the cosmetic composition, 0.05 to 5 wt% of the self-emulsifying agent, 0.01 to 5 wt% of the self-emulsifying stabilizer, and 0.5 to 80 wt% of the oil phase stabilizer.

7. The emulsion cosmetic composition of claim 1, wherein the self-emulsifying agent and the self-emulsifying stabilizer are mixed at a weight ratio of 1:0.002 to 100.

8. The emulsion cosmetic composition of claim 1, which is of an oil-in-water (O/W) type, a water-in-oil (W/O) type, a water-in-oil-in-water (W/O/W) type, or an oil-in-water-in-oil (O/W/O) type.
